# EUROPEAN PATENT SPECIFICATION

(11) **EP 1 809 268 B1**
(45) Date of publication and mention of the grant of the patent: **03.08.2011**
(21) Application number: 04761549.7
(22) Date of filing: 01.10.2004
(51) Int. Cl.: A61K 31/015, A61P 7/10, A61P 29/00

(54) **Alpha-humulene for use in the prophylaxis or treatment of inflammatory pain or oedema**
Alpha-humulene zur Verwendung bei der Prophylaxe oder Behandlung der entzündlichen Schmerzen und des Ödems
Alpha-humulène pour son utilisation dans la prophylaxie ou le traitement de la douleur inflammatoire ou de l'oedème

(43) Date of publication of application: 25.07.2007
(73) Proprietor: Aché Laboratórios Farmacêuticos S.A., 07034-904 Guarulhos - SP (BR)
(72) Inventor: PIANOWSKI, Luiz, F., 12947-150 Atibaia - SP (BR); CALIXTO, João, B., 88037-120 Florianópolis - SC (BR); BRANDÃO, Dagoberto de C., 05016-081 São Paulo - SP (BR)
(74) Representative: Engelhard, Markus
(86) International application number: PCT/BR2004/000189
(87) International publication number: WO 2006/037194

(56) References cited:
- WO-A2-03/020371
- WO-A2-2004/066912
- JP-A- 07 215 846
- US-A1- 2003 008 021

## Description

The present invention concerns the use of caryophyllenes related to medicaments and to the treatment of bodily conditions of inflammation and inflammatory pain. It relates particularly to the use of caryophyllenes in the manufacture of medicaments for the treatment of inflammatory conditions of the animal body, including the human body. The invention also concerns the use of caryophyllenes for the treatment of inflammatory conditions of the body, including inflammatory pain.

Caryophyllenes are known chemical compounds, useful in various applications. For instance patent document US 3,987,008 reveals sesquiterpenic derivatives as odor and taste-modifying agents; In J. Nat. Prod. 1992 Jul;55(7):999-1003, beta-caryophyllene and alpha-humulene as cited as potential anticarcinogenic agents; in patent application WO 9218001 alpha and beta-humulene and (-)-beta-caryophyllene are cited in the control of whitefly species; in patent US5,314,693 alpha-humulene is cited as a repellent for pine wood nematodes; in patent application WO 02078719 alpha and beta-caryophyllene are comprised in antitumor compositions.

The present specification discloses, in one particular aspect, a useful use for caryophyllenes, more particularly alpha-humulene or beta-caryophyllene, as anti-inflammatory and as analgesic, in a broad sense. In a more specific sense, the compounds were found to be useful inhibitors of entities that are known to be involved in the inflammatory process :
- pro-inflammatory cytokines IL-1β (interleukin 1β) and TNFα (tumor necrosis factor α);
- PGE2 (prostaglandin-E2),
- Expression of COX-2 (cycloxigenase-2) and iNOS (inducible nitric oxide synthase) enzymes

One particular example of caryophyllene, which is the caryophyllene of the invention, is alpha-humulene, a sesquiterpene identified with the CAS (Chemical Abstracts Service) registry number 6753-98-6, also known as alpha-caryophyllene, represented by the following structure :

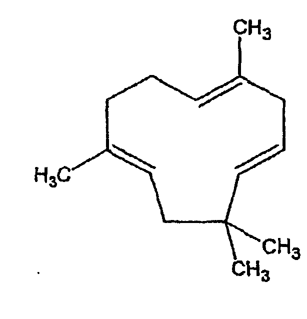

Another particular example of caryophyllene not being part of the invention is the trans-caryophyllene (or beta-caryophyllene), also a sesquiterpene, identified with the CAS (Chemical Abstracts Service) registry number 87-44-5, represented by the following alternative structures A and B:

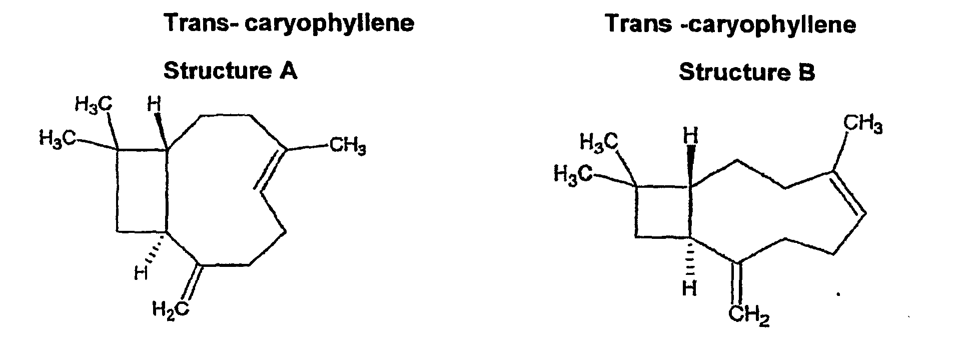

According to the meaning employed herein, mention to caryophyllenes, object of the invention, includes the molecules as such, their salts, isomers, metabolites, pro-drugs, solvates (including hydrates) and adducts.

Terpenes, including sesquiterpenes, are often mentioned as components of complex mixtures extracted from plants, where - as known to a persons skilled in the art - it is undetermined what compound or compounds are effective, how much effective they are, and whether they are active by themselves, by way of the vehicle/solvent the composition contains (water, alcohol, other solvents, mixtures of those, etc), or by way of their interaction with other components within the mixtures. Individual terpenes per se, of natural origin or products of synthesis (for instance J. Am. Chem. Soc., 99, 3864 (1977)), are rarely mentioned as effective pharmaceutical agents. Alpha-humulene has even been mentioned to be virtually inactive concerning anti-inflammatory or chemo-therapic effects (reference: Carcinogenesis (2002), 23(5), 795-802).

The specification discloses that caryophyllenes, particularly alpha-humulene and trans-caryophyllene, have marked anti-inflammatory effects, including inflammatory pain, comprised therein the inhibitory effect upon the production of pro-inflammatory cytokines IL-1β and TNFα, prostaglandin PGE2, or the expression of enzymes COX-2 and iNOS.

The specification further discloses that caryophyllenes, particularly alpha-humulene and trans-caryophyllene, have anti-allergic, particularly antihistaminic effects.

The caryophyllenes disclosed herein are part of the ongoing search for drugs with direct of indirect inflammatory activity, which inhibit the physiopathology processes involved in inflammation. They are used in the control of chronic-degenerative diseases as rheumatoid arthritis, osteoarthritis, systemic lupus eritematosus, ulcerative colitis, psoriasis, atopic eczema, atherosclerosis, and other non degenerative diseases as depression, and cellulites, and allergies.

Therefore, one of the objects of the present invention is the use of alpha-humulene or compositions comprising alpha-humulene, in the manufacture of a medicament for the treatment of inflammatory conditions of the animal body, particularly the human body.

Another object of the present invention is the use of alpha-humulene, or compositions containing alpha-humulene, in the treatment of inflammatory conditions of the animal body, particularly the human body.

Another object of the present invention is a method of treatment of an inflammatory condition of the animal body, particularly the human body, comprising the administration of a therapeutically effective amount of alpha-humulene to a patient.

Further objects of the present invention are defined in the claims.

Also disclosed herein is the use of caryophyllenes, particularly alpha-humulene and trans-caryophyllene, or compositions containing caryophyllenes, for the inhibition of the bodily production of one or more of cytokine IL-1β, cytokine TNFα, prostaglandin PGE2, expression of enzymes COX-2 and iNOS.

The caryophyllene of the invention, i.e. alpha-humulene as well as compositions comprising the caryophyllene according to the invention, can be administered to the subject in need of treatment in any adequate way, enteral or parenteral, including oral, topical, transdermal, subcutaneous, intraperitonial, intravenous, by infiltration, by inhalation, transdermal, transmucosal, intramuscular, intrapulmonary, vaginal, rectal, intraocular, and sublingual. Particularly adequate ways of administration in the present invention are systemically (infiltration, oral, inhalation by spray, transdermal) and topically. The caryophyllene of the invention can be comprised in a slow or controlled release composition. Known adjuvants and excipients can be utilized in the compositions. A reference for pharmaceutical dosage forms useful for the compositions related to the inventions can be found in the publication Remington's Pharmaceutical Sciences, Mack Publishing.

The compositions comprising the caryophyllene of the invention can be administered to patients as solids, liquids or semi-liquids, tablets, capsules, pills, powder, granules, suspensions, emulsions, dispersions and any other useful known form.

The compositions might contain further active agents, for instance antibiotics, depending on the desired effect.

For oral administration as tablets or capsules (both soft and hard capsules), the caryophyllene of the invention can be combined with pharmaceutically acceptable inert vehicles, such as lactose, starch, sucrose, glucose, methyl cellulose, magnesium stearate, dicalcium phosphate, calcium phosphate, manitol, sorbitol, and similars; for oral administration in the liquid form, the caryophyllenes can be combined with ethanol, glycerol, water, and similars. When desired or necessary, agglomerating agents, lubricant agents, disintegrating agents, color and fragrance can be added to the mixture. Common agglomerating agents are glucose, β-lactone, corn sweeteners, natural or synthetic gums such as gum arabica, tragacanth or sodium alginate, carboxymethylcellulose, polyethylene glycol, wax and similars. Lubricants include sodium oleate, sodium stearate, magnesium stearate, sodium benzoate, sodium acetate, sodium chloride. Disintegrants include starch, methyl cellulose, agar, bentonite, xanthan gum, and similars.

The compositions concerned in the invention can also be administrated as liposomes or coupled with soluble polymers as vehicles.

Liquid dosage forms for oral administration may comprise colorants and edulcorants to increase acceptance by patients. Acceptable vehicles for water dosage forms are, water, an appropriate oil, a saline solution, aqueous dextrose, other sugar solutions and glycols as propylene glycol or polyethylene glycols, phosphate buffer.

Compositions related to the present invention typically comprise about 1 mg to about 1000 mg of one or more alpha-humulene, particularly about 10 to 200mg and more particularly about 30 to 100 mg. In such compositions the caryophyllene represents about 0.1 to 99% in weight, particularly about 1 to 70% and more particularly about 10 to 40%, optionally comprising at least one pharmaceutically acceptable vehicle.

### EXAMPLES

The examples that follow represent particular embodiments of the invention, and do not impose any limitation to its extension, which is limited only by the claims attached hereto.

Furthermore, the following examples show experiments using the caryophyllene of the invention, i.e. alpha-humulene. In some cases, trans-caryophyllene has been involved in these experiments for comparison.

### EXAMPLE 1

### INFLAMMATORY NOCICEPTION INDUCED BY CARREGENIN.

The evaluation methodology used in this tests is described by Vaz et al. in J. Pharmacol. Exp. Ther. 278:304-312, 1996.

Male mice (25-35g) were systemically (orally) treated with alpha-humulene, 50 mg/kg, administered 1 hour before the experiment. Animals treated with 0.9% saline solution (0.1ml/10g) were used as the control. Another group of animals was treated with paracetamol (600 mg/kg, orally, administered 1h before the treatment), that was used as positive control. For the induction of inflammatory pain, the animals received an intraplantar injection of 0.05 ml of carrageenin (300 µg per paw) at the plantar surface of the right hind paw. This dosage causes oedema, nociception and substantial swelling of the injected paw.

The nociception was evaluated with a Von Frey filament (0.4g) after 3, 4 and 6 hours. To obtain a basal response, the animals were pre-tested the previous day with the 0.4g von Frey filament. Only animals with a response of about 20% were selected. The filament was applied to the right hind paw, complying with the criteria of (1) the application was perpendicular to the plantar surface, with enough pressure to cause the filament to bend, thus obtaining total pressure; (2) the animals were evaluated when the four paws were touching the screen; (3) the paw withdrawal response was considered when the animal removed the paw entirely from the support screen; (4) each animal

the caryophyllene represents about 0.1 to 99% in weight, particularly about 1 to 70% and more particularly about 10 to 40%, optionally comprising at least one pharmaceutically acceptable vehicle.

### EXAMPLES

The examples that follow represent particular embodiments of the invention, and do not impose any limitation to its extension, which is limited only by the claims attached hereto.

### EXAMPLE 1

### INFLAMMATORY NOCICEPTION INDUCED BY CARREGENIN.

The evaluation methodology used in this tests is described by Vaz et al. in J. Pharmacol. Exp. Ther. 278:304-312, 1996.

Male mice (25-35g) were systemically (orally) treated with alpha-humulene, 50 mg/kg, administered 1 hour before the experiment. Animals treated with 0.9% saline solution (0.1ml/10g) were used as the control. Another group of animals was treated with paracetamol (600 mg/kg, orally, administered 1h before the treatment), that was used as positive control. For the induction of inflammatory pain, the animals received an intraplantar injection of 0.05 ml of carrageenin (300 µg per paw) at the plantar surface of the right hind paw. This dosage causes oedema, nociception and substantial swelling of the injected paw.

The nociception was evaluated with a Von Frey filament (0.4g) after 3, 4 and 6 hours. To obtain a basal response, the animals were pre-tested the previous day with the 0.4g von Frey filament. Only animals with a response of about 20% were selected. The filament was applied to the right hind paw, complying with the criteria of (1) the application was perpendicular to the plantar surface, with enough pressure to cause the filament to bend, thus obtaining total pressure; (2) the animals were evaluated when the four paws were touching the screen; (3) the paw withdrawal response was considered when the animal removed the paw entirely from the support screen; (4) each animal was stimulated 10 consecutive times, each stimulation lasting 1 second; (5) each paw withdrawal event was considered as 10% of the response, with 10 withdrawal events corresponding to 100% response.

Graph 1 below compares the pain inhibition obtained by alpha-humulene with the administration of paracetamol. Each point represents the average of 5 animals, and the vertical bars the mean standard error deviation.

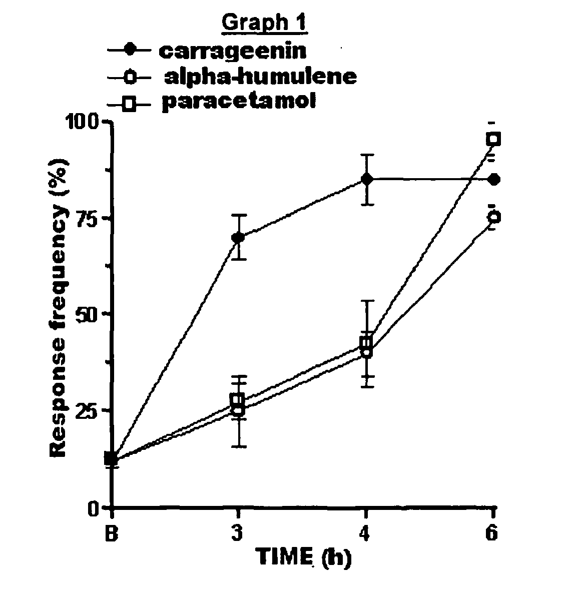

The graph clearly shows that, according to the invention, a caryophyllene as alpha-humulene reduced the inflammatory nociception, as a result of reduction of inflammation, as much as a known analgesic, paracetamol.

### EXAMPLE 2

### CARRAGEENIN OEDEMA IN MOUSE PAW

The test used below is described by Cunha et. al. in the publication Life Sci.70:159-169, 2001.

Male 25g-35g mice were slightly sedated with ether and were injected 50 µl saline containing carrageenin (300 µl/paw) in the right paw. The left paw received the same volume of saline and was taken as a negative control. The swelling was measured with a plethysmometer (manufacturer: Ugo Basile, Italy) along various time intervals after the injection of the phlogistic agent. The difference between the volumes of the right and the left paw were quantified (in ml) and taken as an index of oedema. One hour before the test the animals were systemically treated (orally) with 50 mg/kg of alpha-humulene or trans-caryophyllene.

Graph 2 below compares the inhibition of the volume of the oedema by administration of either alpha-humulene or trans-caryophyllene with inhibition obtained with the administration of dexamethasone (0.5 mg/kg, injected subcutaneously 4h before test) and used as positive control. Oedema volume measurement time point intervals were 30, 60, 120 and 240 min, 24h and 48h. Each point represents the average of 5 animals, and the vertical bars the mean standard error deviation.

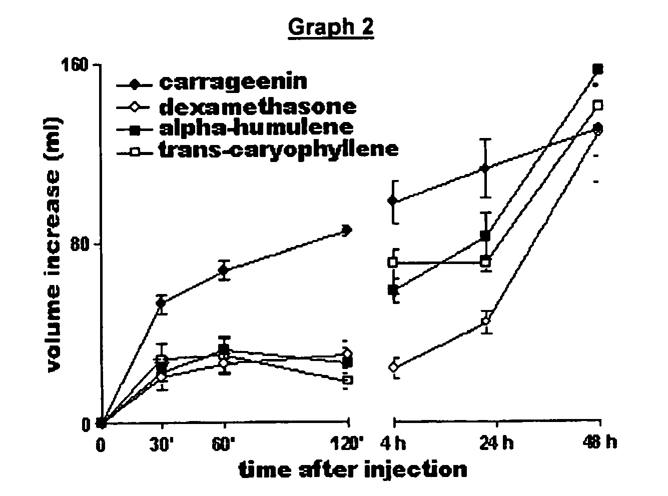

The graph clearly shows that, according to the invention, a caryophyllene as alpha-humulene reduced the inflammatory volume, as did dexamethasone.

### EXAMPLE 3

### BRADYKININ OEDEMA IN MOUSE PAW

The test used below is described by Cunha et. al. in the publication Life Sci.70:159-169, 2001.

Male 25g-35g mice were slightly sedated with ether and were injected 50 µl saline containing bradykinin (BK, 3 nmol/paw), intraplantar, in the right paw. The left paw received the same volume of saline and was taken as negative control. The swelling was measured with a plethysmometer (manufacturer: Ugo Basile, Italy) along various time points intervals after the injection of the phlogistic agent. The difference between the volumes of the right and the left paw were quantified (in ml) and taken as an index of oedema. One hour before the test the animals were systemically treated (orally) with 50 mg/kg of alpha-humulene or with trans-caryophyllene.

The animals were pre-treated with 5 mg/kg of captopril, injected subcutaneously, 1 hour before the test, in order to avoid degradation of kinines.

Graphs 3A and 3B below compare the inhibition of the volume of the oedema by administration of alpha-humulene (3A) or trans-caryophyllene (3B). Oedema volume measurement time intervals were 10, 20, 30, 60, and 120 min, 24h and 48h. Each point represents the average of 5 animals, and the vertical bars the mean standard error deviation.

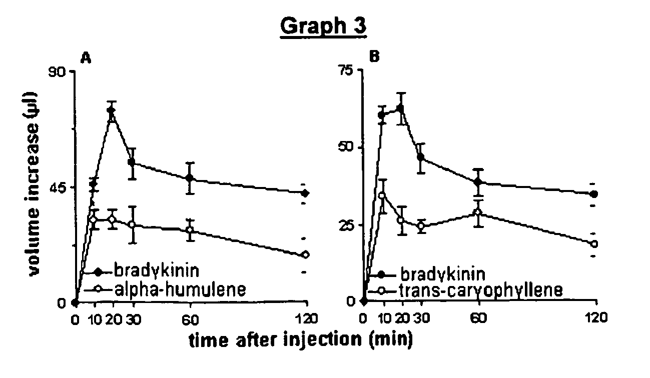

The graph clearly shows that, according to the invention, a caryophyllene as alpha-humulene or trans-caryophyllene markedly reduced bradykinin-induced paw oedema.

### EXAMPLE 4

### HISTAMINE OEDEMA IN MOUSE PAW

The test used below is described by Cunha et. al. in the publication Life Sci.70:159-169, 2001.

Male 25g-35g mice were slightly sedated with ether and were injected 50 µl saline containing histamine (100 nmol/paw), intraplantar, in the right paw. The left paw received the same volume of saline and was taken as negative control. The swelling was measured with a plethysmometer (manufacturer: Ugo Basile, Italy) along various time intervals after the injection of the phlogistic agent. The difference between the volumes of the right and the left paw were quantified (in ml) and taken as an index of oedema. One hour before the test the animals were systemically treated (orally) with 50 mg/kg of alpha-humulene.

Graph 4 below compares the inhibition of the volume of the oedema by administration of alpha-humulene. Oedema volume measurement time intervals were 10, 20, 30, 60, and 120 min, 24h and 48h. Each point represents the average of 5 animals, and the vertical bars the mean standard deviation.

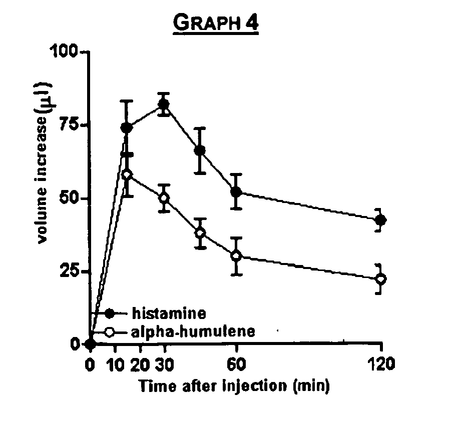

The graph clearly shows that, according to the invention, a caryophyllene as alpha-humulene significantly reduced histamine-induced oedema formation. It also indirectly shows effect against allergy.

### EXAMPLE 5

### PLATELET AGGREGATION FACTOR (PAF) OEDEMA IN MOUSE PAW

The test used below is described by Cunha et. al. in the publication Life Sci.70:159-169, 2001.

Male 25g-35g mice were slightly sedated with ether and were injected 50 µl saline containing platelet aggregation factor (PAF, 3nmol/paw), intraplantar, in the right paw. The left paw received the same volume of saline and was taken as negative control. The swelling was measured with a plethysmometer (manufacturer: Ugo Basile, Italy) along various time intervals after the injection of the phlogistic agent. The difference between the volumes of the right and the left paw were quantified (in ml) and taken as an index of oedema. One hour before the test the animals were systemically treated (orally) with 50 mg/kg of alpha-humulene or trans-caryophyllene.

Graph 5 below compares the inhibition of the volume of the oedema by administration of alpha-humulene (5A) and trans-caryophyllene (5B). Oedema volume measurement time intervals were 30, 45, 60, and 120 min. Each point represents the average of 5 animals, and the vertical bars the mean standard deviation.

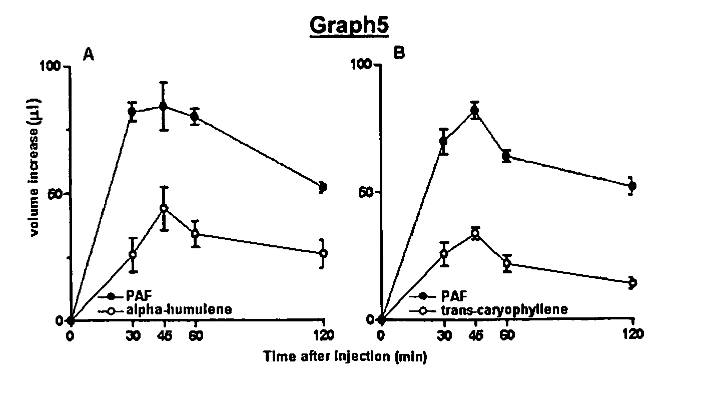

The graph clearly shows that, according to the invention, a caryophyllene as alpha-humulene or trans-caryophyllene markedly reduced PAF-induced oedema formation. As PAF is also known to be involved in allergic processes, such data further reinforces the use of caryophyllenes in the management of allergic states.

### EXAMPLE 6

### ARACHIDONIC ACID OEDEMA IN MOUSE EAR

The ear oedema in the test below was measured according to Calixto et. al. in the publication Prostaglandins, 5: 515 - 526, 1991, with minor modifications.

Male 25g-35g mice, in a first group, were topically applied, in the inner surface of the ears, an ointment comprising a range from 0.025 to 0.2% alpha-humulene or trans-caryophyllene. In the positive control group, the animals were topically applied 0.05 mg of phenidone per ear. After 60 minutes, the animals received 20 µl of arachidonic acid (2 mg/ear), dissolved in acetone, in the inner surface of the right ear. The oedema was measured using a digital micrometer, and the responses were expressed as µm, the difference between the ear thickness before and after the application of arachidonic acid. The responses of the animals treated with caryophyllenes were compared to those observed in the control group animals, treated with base ointment.

Graphs 6A and 6B below compare the inhibition of the volume of the oedema by topic administration of alpha-humulene (6A) and trans-caryophyllene (6B). Oedema volume measurement was performed after application of 0.025%, 0.05%, 0.1, and 0.2% caryophyllene content ointment, compared to the oedema volume caused by the application of phenidone and arachidonic acid (C). Each point represents the average of 5 animals, and the vertical bars the mean standard error deviation.

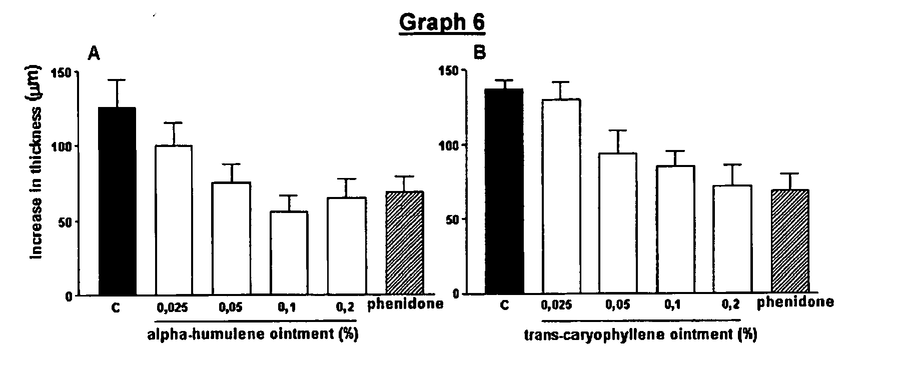

The graphs clearly show that, according to the invention, the topic application of a caryophyllene as alpha-humulene or trans-caryophyllene markedly reduced oedema formation, in a dose-dependent manner.

### EXAMPLE 7

### LEVELS OF PRO-INFLAMMATORY CYTOKINE IL-1B

The test used below is described by Campos et. al. in the publication Br. J. Pharmacol. 135: 1107-1114, 2002, with minor modifications.

Male 160-180g rats were orally given 50 mg/kg of alpha-humulene. Animals treated with 0.9% (0.1 ml/10g) saline were used as control. Another group of animals was treated with 0.5 mg/kg dexamethasone, subcutaneously, 4 hours before the test, and used as positive control. After 60 minutes, the animals received intraplantar injections of 100 µl of carrageenin (300 µg/paw) and were sacrificed after 180 minutes. Control animals received saline. The subcutaneous tissue of the injected paws was removed and put in a phosphate buffer containing 0.5% tween 20, 0.1 mM benzametonium chloride, 10 mM EDTA, 2µg/m aprotinin, 0,1 mM PMSF (phenyl methyl sulfonyl fluoride) and 0,5 % BSA (bovine serum albumin). The tissues were homogenized and centrifuged at 3000 g, for 10 min, at - 4 °C. The supernatant was used in the test. The levels of IL-1β were measures with an Elisa kit, according to the manufacturer's instructions (R & D Systems ®, USA). The tests were performed in duplicate, and repeated three times. The answers are expressed in µg/mg of tissue.

Graph 7 below (each result represents the average of 5 animals, and the vertical bars the mean standard error deviation) compares the inhibition of production of inflammatory cytokine IL-1β induced by carrageenin in the paws of rats.

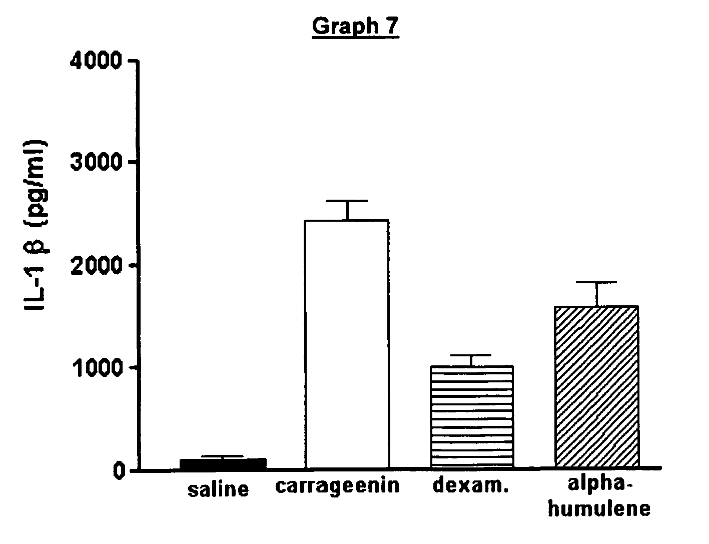

The graph clearly shows that, according to the invention, the administration of a caryophyllene, such as alpha-humulene, markedly inhibited the production of pro-inflammatory cytokine IL-1β induced by carrageenin in the paws of rats.

### EXAMPLE 8

### LEVELS OF PRO-INFLAMMATORY CYTOKINE TNFα

The test used below is described by Campos et. al. in the publication Br. J. Pharmaco/. 135: 1107-1114, 2002, with minor modifications.

Male 160-180g rats were orally given 50 mg/kg of trans-caryophyllene. Animals treated with 0.9% (0.1 ml/10g) saline were used as control. Another group of animals was treated with 0.5 mg/kg dexamethasone, subcutaneously, 4 hours before the test, and used as positive control. After 60 minutes, the animals received intraplantar injections of 100 µl of carrageenin (300 µg/paw) and were sacrificed after 180 minutes. Control animals received saline. The subcutaneous tissue of the injected paws was removed and put in a phosphate buffer containing 0.5% tween 20, 0.1 mM benzametonium chloride, 10 mM EDTA, 2µg/m aprotinin, 0,1 mM PMSF (phenyl methyl sulfonyl fluoride) and 0,5 % BSA (bovine serum albumin). The tissues were homogenized and centrifuged at 3000 g, for 10 min, at - 4 °C. The supernatant was used in the test. The levels of TNFα were measures with an Elisa kit, according to the manufacturer's instructions (R & D Systems ®, USA). The tests were performed in duplicate, and repeated three times. The answers are expressed in pg/mg of tissue.

Graph 8 below (each result represents the average of 5 animals, and the vertical bars the mean standard error deviation) compares the inhibition of production of inflammatory cytokine TNFα induced by carrageenin in the paws of rats.

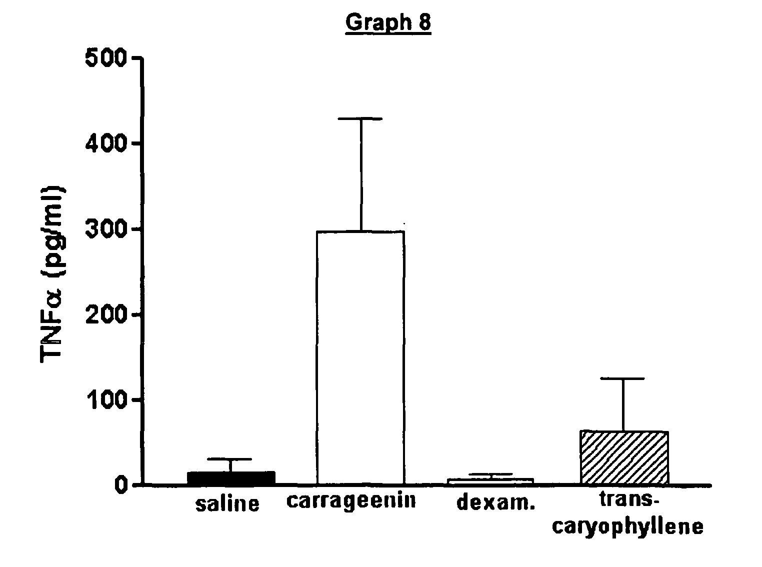

The graph clearly shows that, according to the invention, the administration of a caryophyllene, such as trans-caryophyllene, markedly inhibited the production of the pro-inflammatory cytokine TNFα induced by carrageenin in the paws of rats.

### EXAMPLE 9

### LEVELS OF PGE2

The test used below is described by Pinheiro et. al. in the publication Inflamm. Res. 51: 603- 610, 2002, with minor modifications.

Male 160-180g rats were orally given 50 mg/kg of trans-caryophyllene. Animals treated with 0.9% (0.1 ml/10g) saline were used as control. Another group of animals was treated with 0.5 mg/kg dexamethasone, subcutaneously, 4 hours before the test, and used as positive control. After 60 minutes, the animals received intraplantar injections of 100 µl of carrageenin (300 µg/paw) and were sacrificed after 180 minutes.

The exsudate of the paws was collected by dialysis with the help of two polyethylene canulas, and was utilized for the quantification of PGE2, with an Elisa kit, according to the manufacturer's instructions (R & D Systems ®, USA). The tests were performed in duplicate, and repeated three times. The answers are expressed in pg/mg of tissue.

Graph 9 below (each result represents the average of 5 animals, and the vertical bars the mean standard error deviation) compares inhibition of the PGE2 level growth induced by carrageenin in the paws of rats, by administration of alpha-humulene and trans-caryophyllene, compared to the effect obtained by treatment with dexomethasone.

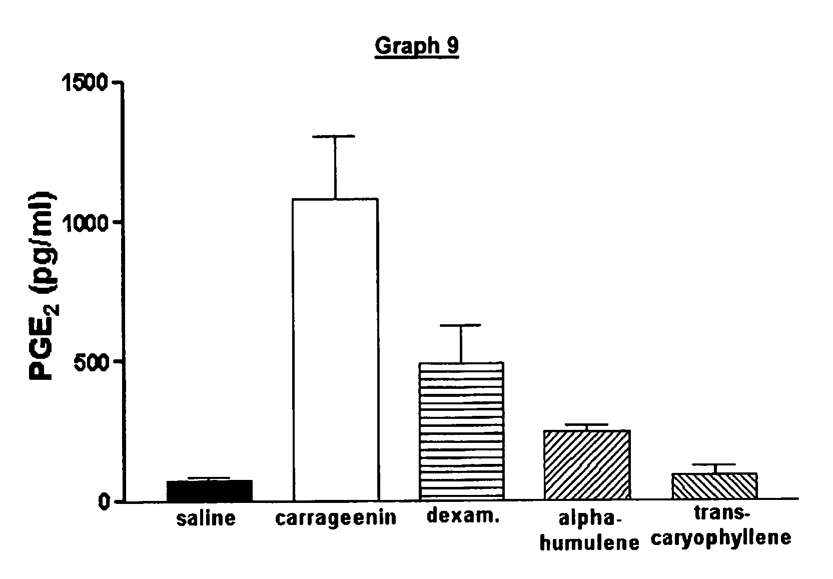

The graph clearly shows that, according to the invention, the administration of a caryophyllene, such as alpha humulene or trans-caryophyllene, markedly inhibited the growth of PGE2 levels induced by carrageenin in the paws of rats.

### Example 10

### Inhibition of the expression of enzymes COX-2 and iNOS

The expression of enzymes COX-2 and iNOS were determined by Western blot according to the methodology described by Medeiros et. al. in the publication Circ Res. 28:1375 - 1382, 2004.

Male 160-180g rats were orally given 50 mg/kg of trans-caryophyllene. Animals treated with 0.9% (0.1 ml/10g) saline were used as control. Another group of animals was treated with 0.5 mg/kg dexamethasone, subcutaneously, 4 hours before the test, and used as positive control. After 60 minutes, the animals received intraplantar injections of 100 µl of carrageenin (300 µg/paw) and were sacrificed after 180 minutes, and subcutaneous paw tissue was removed 240 minutes after the carrageenin injection.

The collected tissue was immediately frozen in liquid nitrogen and re-suspended in a buffer of hypotonic lysis (10mM HEPES N-2-hydroxyethylpiperazine-N'-2-ethanesulfonic acid, 1.5mM MgCl2, 10mM KCI, 0.5mM PMSF phenylmethylsulphonyl fluoride, 1.5 µg/ml trypsin inhibitor, 7 µg/ml pepstatin A, 5 µg/ml leupeptin, 0.1 mM benzamidine 0,1 mM and 0.5 mM dithiothreitol) and homogenized. The homogenate was divided in three 2ml aliquots, cooled in ice for 15 minutes, vigorously agitated and once again cooled in ice, in the presence of 20 µl 10% non-ionic detergent Nonidet P-40 (Roche Diagnostics, USA).The nuclear fraction was precipitated by centrifugation (1,500 g, 5 minutes) and the supernatant containing the cytosolic extract was stored at -70°C for the Western blot tests. The protein concentration was determined by the Bradford method (BioRad Laboratories Inc. kit, Milan, Italy). The extracts were boiled with v/v equivalent amounts of Laemmly buffer (125 mM of Tris-HCl, 2 mM of EDTA, 4 % of dodecyl sodium sulphate, 20 % of glycerol, 10 % of 2-mercaptoethanol and 0,1 % of Comassie brilliant blue, pH 6.8). The proteins were transferred to nitrocellulose membranes (100 µg/well) and separated by electrophoresis. The membranes were later blocked by *overnight* incubation (4 °C) with skimmed powder milk (10 % PBS), and then incubated with the anti-iNOS or anti-COX-2 antibodies for 1 h at room temperature. The membranes were washed three times with 10% Triton-X in PBS with the antibody peroxidase conjugated (anti-rabbit). The bands thus obtained were quantified using a chemoluminescence kit and densitometry analysis (relative units) in radiographic films.

Graph 10 below (each result represents the average of 5 animals, and the vertical bars the mean standard error deviation) compares inhibition of expression of the COX2 enzymes, obtained by the administration of alpha-humulene and trans-caryophyllene, when evaluated in the subcutaneous tissue or the paw injected with carrageenin, compared to the expression of COX2 induced by carrageenin obtained by treatment with dexomethasone.

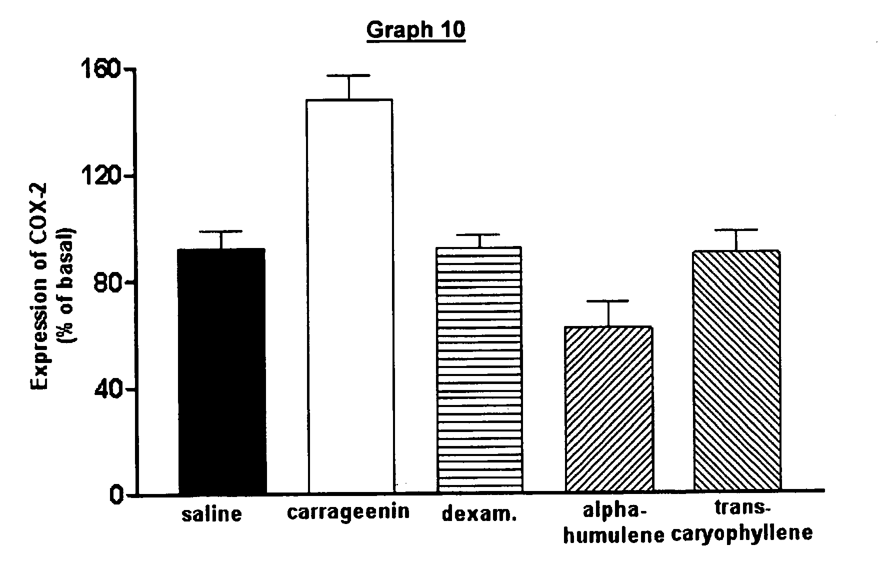

The graph clearly shows that, according to the invention, the administration of a caryophyllene, such as alpha humulene or trans-caryophyllene, markedly inhibited the expression of enzymes COX2 induced by carrageenin in the paws of rats.

Graph 11 below (each result represents the average of 5 animals, and the vertical bars the mean standard error deviation) compares inhibition of expression of the iNOS enzymes, obtained by the administration of alpha-humulene, when evaluated in the subcutaneous tissue or the paw injected with carrageenin, compared to the expression of iNOS induced by carrageenin obtained by treatment with dexomethasone.

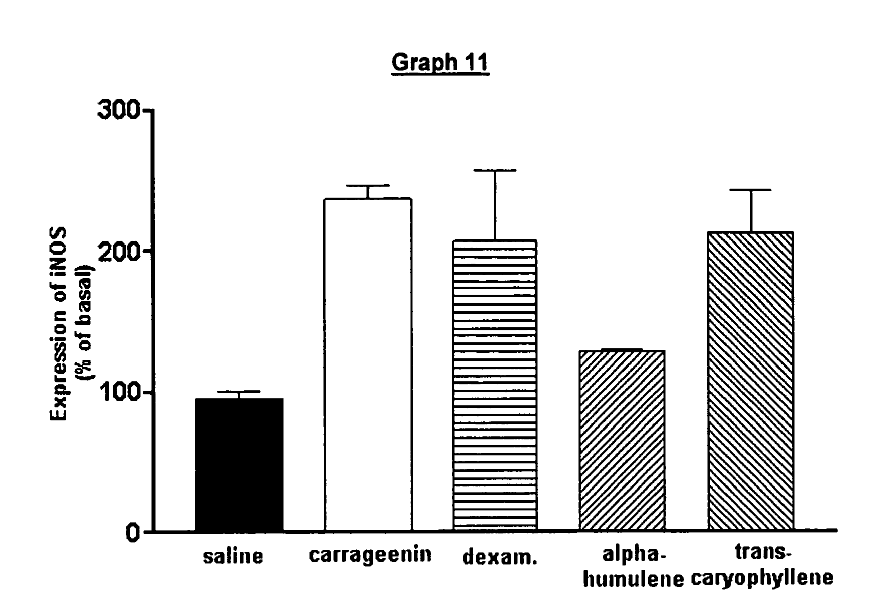

The graph clearly shows that, according to the invention, the administration of a caryophyllene, such as alpha humulene, markedly inhibited the expression of enzymes iNOS induced by carrageenin in the paws of rats.

The examples and the information provided herein concern particular embodiments of the present invention, which is only limited by the breath of the claims attached hereto.

## Claims

1. An alpha-humulene for use in the prophylaxis or treatment of an inflammatory condition of an animal body, wherein the inflammatory condition is inflammatory pain or oedema.

2. The alpha-humulene for use according to claim 1, wherein the animal body is a human body.

3. The alpha-humulene for use according to claim 1 or 2 for an enteral or parenteral administration selected from the group consisting of oral, topical, transdermal, subcutaneous, intraperitoneal, intravenous, transdermal, transmucosal, intramuscular, intrapulmonary, vaginal, rectal, intraocular and sublingual administration, and administration by infiltration or inhalation.

4. The alpha-humulene for use according to claim 1 or 2 for topical or systemic administration selected from the group consisting of oral or transdermal administration, and administration by infiltration or inhalation.

5. A pharmaceutical composition comprising an alpha-humulene as the only active agent having an anti-inflammatory effect.

6. The pharmaceutical composition according to claim 5, comprising from 0.1 to 99% by weight, preferably from 1 to 70% by weight, and most preferably from 10 to 40% alpha-humulene by weight.

7. The pharmaceutical composition according to claim 6, comprising from 1 to 1000 mg, preferably from 10 to 200 mg, and most preferably from 30 to 100 mg alpha-humulene.

8. The pharmaceutical composition according to claim 5 which is a pharmaceutical ointment, wherein the ointment comprises from 0.025 to 0.2% alpha-humulene.

## Patentansprüche

1. Ein alpha-Humulen zur Verwendung bei der Prophylaxe oder der Behandlung eines entzündlichen Zustandes eines tierischen Körpers, wobei es sich bei dem entzündlichen Zustand um Entzündungsschmerz oder ein Ödem handelt.

2. Alpha-Humulen zur Verwendung nach Anspruch 1, wobei es sich bei dem tierischen Körper um einen menschlichen Körper handelt.

3. Alpha-Humulen zur Verwendung nach Anspruch 1 oder 2 zur enteralen oder parenteralen Verabreichung, ausgewählt aus der Gruppe bestehend aus oraler, topischer, transdermaler, subkutaner, intraperitonealer, intravenöser, transdermaler, transmukosaler, intramuskulärer, intrapulmonärer, vaginaler, rektaler, intraokularer und sublingualer Verabreichung, und Verabreichung per Infiltration oder Inhalation.

4. Alpha-Humulen zur Verwendung nach Anspruch 1 oder 2 zur topischen oder systemischen Verabreichung, ausgewählt aus der Gruppe bestehend aus oraler oder transdermaler Verabreichung, und Verabreichung per Infiltration oder Inhalation.

5. Eine pharmazeutische Zusammensetzung, welche ein alpha-Humulen als einzigen Wirkstoff mit einer entzündungshemmenden Wirkung umfasst.

6. Pharmazeutische Zusammensetzung nach Anspruch 5, welche 0,1 bis 99 Gewichtsprozent, vorzugsweise 1 bis 70 Gewichtsprozent, und am bevorzugtesten 10 bis 40 Gewichtsprozent alpha-Humulen umfasst.

7. Pharmazeutische Zusammensetzung nach Anspruch 6, welche 1 bis 1000 mg, vorzugsweise 10 bis 200 mg, und am bevorzugtesten 30 bis 100 mg alpha-Humulen umfasst.

8. Pharmazeutische Zusammensetzung nach Anspruch 5, welche eine pharmazeutische Salbe ist, wobei die Salbe 0,025 bis 0,2 % alpha-Humulen umfasst.

## Revendications

1. Utilisation d'alpha-humulène pour la prophylaxie ou le traitement d'une affection inflammatoire d'un corps animal, l'affection inflammatoire étant une douleur inflammatoire ou un oedème.

2. Utilisation d'alpha-humulène selon la revendication 1, dans lequel le corps animal est un corps humain.

3. Utilisation d'alpha-humulène selon la revendication 1 ou 2, pour une administration entérale ou parentérale choisie dans le groupe comprenant une administration orale, topique, transdermique, sous-cutanée, intrapéritonéale, intraveineuse, transdermique, transmuqueuse, intramusculaire, intrapulmonaire, vaginale, rectale, intra-oculaire et sublinguale, et une administration par infiltration ou par inhalation.

4. Utilisation d'alpha-humulène selon la revendication 1 ou 2, pour l'administration topique ou systémique choisie dans le groupe comprenant l'administration orale ou transdermique et l'administration par infiltration ou inhalation.

5. Composition pharmaceutique comprenant un alpha-humulène comme unique principe actif ayant un effet anti-inflammatoire.

6. Composition pharmaceutique selon la revendication 5, comprenant de 0,1 à 99 % en poids, de préférence de 1 à 70 % en poids et plus préférentiellement, de 10 à 40 % en poids d'alpha-humulène.

7. Composition pharmaceutique selon la revendication 6, comprenant de 1 à 1000 mg, de préférence de 10 à 200 mg et plus préférentiellement, de 30 à 100 mg d'alpha-humulène.

8. Composition pharmaceutique selon la revendication 5, qui est une pommade pharmaceutique, la pommade comprenant de 0,025 à 0,2 % d'alpha-humulène.
